# EUROPEAN PATENT APPLICATION

(11) **EP 1 350 853 A1**
(43) Date of publication of application: **08.10.2003**
(21) Application number: 02076336.3
(22) Date of filing: 05.04.2002
(51) Int. Cl.: C12Q 1/68

(54) **Detection of polymorphisms**

(71) Applicant: ID-Lelystad, Instituut voor Dierhouderij en Diergezondheid B.V., 8219 PH Lelystad (NL)
(72) Inventor: Agbo, Edwin Chukwura, 8212 BE Lelystad (NL); te Pas, Marinus Frederick Willem, 3882 XG Putten (NL); Smits, Marinus Adrianus, 3844 KE Harderwijk (NL)
(74) Representative: Prins, Adrianus Willem

(57) **Abstract**

The present invention provides methods for producing a nucleic acid fingerprint as well as methods for detecting sequence polymorphisms between one or more genomes. The methods involve: (a) fragmenting a nucleic acid into nucleic acid fragments with ends that are compatible to ligation with at least one adapter, (b) performing a ligation reaction between the compatible ends of the nucleic acid fragments and at least one adapter, (c) amplifying the nucleic acid fragments by using at least one amplification primer, and (d) generating a nucleic acid fingerprint from the amplified fragments. A method according to the present invention permits high-resolution fingerprinting while maintaining stringency in a PCR reaction. In another aspect, the invention also provides a kit for preparing nucleic acid fingerprints according to methods of the invention.

## Description

### Field of the invention

The invention relates to the field of genetic diversity analysis. More particularly, the invention relates to the genetic analysis of nucleotide sequence polymorphisms in DNA by nucleic acid fingerprinting.

### Background of the invention

In order to assess genetic diversity among prokaryotic or eukaryotic organisms and to address questions regarding population structure and genetic relatedness, a variety of methods, predominantly genome fingerprinting methods, has been developed in recent years (see Mueller and Wolfenbarger for review).

Methods such as genome fingerprinting have been developed with the aim of providing an estimate, preferably essentially unbiased, of the total genome variance in a population of organisms, while minimizing errors due to sampling variance.

The amplified fragment length polymorphism (AFLP) method (Vos *et al.* 1995), for example, has shown great value for interspecies genotyping, and has been reported to offer low resolution in the typing of, for example, trypanosome subspecies (Agbo *et al*., 2002) and closely related strains in other taxa (Lindstedt *et al*., 2000).

AFLP is a DNA fingerprinting method that detects defined DNA restriction fragments by means of PCR amplification. The method comprises the steps of a) digesting DNA with restriction endonuclease enzymes, b) ligating double stranded (ds) adapters to the ends of the restriction fragments to which PCR primers can be annealed, c) amplifying a subset of the restriction fragments using two primers essentially complementary to the adapter, d) separating the amplified restriction fragments on denaturing polyacrylamide gels by gel-electrophoresis and e) visualizing the separated restriction fragments by means of autoradiography, phospho-imaging, or other methods, thus obtaining a DNA fingerprint. Essentially, AFLP accesses subsets of polymorphisms at one or two restriction sites.

For most living organisms, except the smaller viruses, restriction digestion of the genomic DNA, or nucleic acid such as cDNA derived there from, generally results in such a large number of fragments that full separation into discrete fragments on a gel is no longer feasible. Therefore, often only a small fraction of the DNA fragments are selected to be detected. This selection for example occurs during PCR amplification or sometimes even haphazardly during visualization or detection on, for example, a gel. In case the selection occurs during PCR amplification this is usually done by preselecting a subset of tagged restriction fragments, which pre-selection resides in the specific design of the oligonucleotides that are used as primers in the PCR amplification reaction, so that only a relatively small number of tagged restriction fragments will be amplified during the PCR amplification reaction.

As mentioned, in many applications, such as the typing of species, conventional DNA fingerprinting methods provide sufficient sensitivity to detect differences within otherwise identical DNA fingerprints, also known as DNA polymorphisms. Polymorphic positions in the DNA can be a suitable basis for the development of DNA markers.

However, in the case of comparative analysis of genomes of even more closely related organisms, wherein relatively little genetic variation is expected, such as the genetic variation within a subspecies, the genetic variation cannot always be revealed by using conventional genetic diversity analysis techniques. In such cases, methods capable of providing increased resolution power are needed in order to reveal nucleotide polymorphisms.

In order to accomplish such increased discriminatory power it is desirable to achieve an even greater resolution of identifiable polymorphic fragments, i.e. to generate more fragments that potentially contain polymorphisms and to detect these in a robust way.

Whereas conventional fingerprinting methods are generally able to detect polymorphisms in the target sites of the restriction endonucleases applied in the DNA digestion reaction as well as polymorphisms in the adjacent nucleotide sequences, by virtue of the selective design of the PCR primers, they are not able to detect polymorphisms in other regions of the DNA fragments produced.

Several attempts have been made to generate more fragments that potentially contain polymorphisms and to detect these, for example, by using more restriction enzymes in one reaction for DNA digestion. Simons and co-workers (Simons *et al*., 1997) pioneered early attempts at the use of additional endonucleases in AFLP to gain access to more independent restriction sites in order to increase the discriminatory power of the method, but reported only slight improvement in fingerprint patterns compared to the standard procedure in which two restriction endonucleases are used.

Lindstedt and co-workers (Lindstedt *et al*., 2000) digested DNA with a set of three restriction endonuclease enzymes followed by adapter ligation and subsequent amplification by using a set of three primers. This method inherently compromises the stringency of the PCR amplification reaction, which may lead to variable results, and compromises the robustness of the method.

More recently, the use of three endonuclease enzymes in a genome fingerprinting method (Van der Wurff *et al*., 2000) was even reported to be very suitable for providing a lower number of potentially amplifiable fragments thus providing a method for studying relatedness between more distantly related taxa.

In certain instances, such as in the case of genetic analysis of organisms to subspecies level, additional discriminatory power is however required. For example, within parasites such as trypanosomes these problems are often exemplified.

Trypanosomes are unicellular, protozoan parasites that cause debilitating disease - collectively called trypanosomosis (Kassai, 1988) - in man and animals. The disease is often fatal to man and animal. *Trypanosoma brucei* consists of three subspecies, which are indistinguishable by conventional morphological, biochemical and antigenic criteria, but differ in their geographical distribution and host specificity (Gibson *et al*., 1980).

*Trypanosoma b. brucei* is one of the trypanosomes that cause 'nagana' in cattle, but does not cause disease in humans, because this subspecies is lysed by normal human serum. *Trypanosoma b. gambiense* and *T. b. rhodesiense* appear to differ in the mechanism of serum resistance (Hawking, 1973; Hawking, 1977). Agbo and co-workers have reported limited associations between different genetic marker loci and resistance in molecular signatures in human- and nonhuman-infective *T. brucei* isolates (Agbo *et al*., 2001). These and other studies have shown an urgent need for additional discriminatory power in fingerprinting techniques.

### Summary of the invention

The invention provides a method wherein a nucleic acid may now be fragmented by any suitable process of nucleic acid fragmentation and wherein nucleic acid fragments are provided that are selectively ligatable to a pair of adapters such that a specific amplification of the nucleic acid fragments is allowed and wherein a nucleic acid fingerprint is generated from the amplified nucleic acid fragments.

The present inventors have now surprisingly found that polymorphisms, preferably in complex and closely-related subspecies may be detected by selecting specific adapter pairs, making the method suitable in a method that fragments nucleic acid in an unspecific manner. The invention provides a method that allows for the use of highly degenerative nucleic acid fragmentation methods in nucleic acid fingerprinting techniques.

The present invention provides a method for producing a nucleic acid fingerprint and comprises the step of providing from a starting nucleic acid a plurality of adapter ligatable nucleic acid fragments with ends that are compatible to at least one adapter, and wherein said nucleic acid fragments are obtained by fragmentation of said starting nucleic acid.

Of course, for fragmentation a random fragmentation or, alternatively, a specific fragmentation may be used. Random fragmentation may comprise the physical shearing of nucleic acids, while specific fragmentation may comprise such methods as enzymatic digestion. In preferred embodiment, in order to get the most defined fragments, an endonuclease enzyme is used in a specific fragmentation procedure.

A method for producing a nucleic acid fingerprint according to the invention further comprises the step of performing a ligation reaction between said ends of said nucleic acid fragments and said at least one adapter such as to produce adapter-ligated nucleic acid fragments. It is the specific choice of the adapters that allows for the use of any method of fragmentation of the nucleic acid in a method according to the present invention.

As long as the adapters allow for the performance of a next step in a method for producing a nucleic acid fingerprint according to the invention which comprises amplifying said adapter-ligated nucleic acid fragments by using at least one amplification primer essentially complementary to the nucleotide sequence of said at least one adapter. Such an amplification reaction may comprise any suitable DNA amplification reaction known to the person skilled in the art such as a PCR amplification reaction.

Finally, a method for producing a nucleic acid fingerprint according to the invention comprises the step of generating from said amplified adapter-ligated nucleic acid fragments a nucleic acid fingerprint.

A method according to the invention comprising the random fragmentation of starting nucleic acid is particularly useful for analyzing DNA of parasites, which often contain very substantial amounts (often >80%) of A+T-rich, non-coding sequences - mainly consisting of repetitive sequences of micro-satellite DNA. On the basis of this method, a pair of adapter can now readily be designed for robust amplification of fragments generated.

A method according to the invention accesses additional independent restriction sites per analysis in comparison to the methods of the prior art. A method according to the invention permits for example the simultaneous use of three, four or more endonucleases thereby providing greater resolution of identifiable polymorphic fragments, in combination with only one pair of adapters and cognate primers to maintain stringency in PCR amplification.

In one embodiment, a method according to the invention makes use of the fact that some endonucleases create cohesive ends that are similar to the overhanging or recessive ends created by other endonucleases. As a result, both restriction sites are compatible (in the sense of Watson-Crick base pairing) with one single adapter whereas they originate from different restriction site sequences. Therefore, a single adapter can be ligated to the overhanging or recessive ends created by two different restriction enzymes.

In another embodiment according to the invention, the DNA is fragmented using unspecific methods for fragmentation and ligated to two adapters followed by selective amplification of DNA fragments.

A method of the invention may also comprise the cleavage or cutting of DNA into fragments by using specific methods of fragmentation such as an S1 nuclease digestion, which may specifically digest A+T-rich DNA sequences of non-coding regions of a genome. As long as the selective adapters allow for the specific amplification of the nucleic acid fragments, any fragmentation method is feasible according to the invention.

In a preferred embodiment of the present invention, the DNA is fragmented using specific methods for fragmentation such as by using restriction enzymes to digest the DNA to be studied into a large number of restriction fragments. The use of, for example, a set of four restriction enzymes provides discrimination at extra sites within the DNA, while only one pair of adapters and cognate primers is required as a direct result of which stringency in a PCR amplification reaction may be maintained.

Such embodiments of the present invention provides enhanced discriminatory power over conventional fingerprinting methods in the analysis of, for example, genetic differences among closely related parasite isolates, such as in trypanosomes.

A method according to the invention may comprise the use of two, three, four or even more restriction endonuclease enzymes thereby allowing for the generation of varying numbers of identifiable polymorphic fragments while at the same time allowing for the use of only one pair of adapters and corresponding amplification primers thereby maintaining the possibility to provide robust PCR reaction conditions.

By using a method according to the present invention, the inventors have been able to characterize a large number of trypanosome isolates to the subspecies level, and demonstrated the greater fingerprinting power of this approach over a well-established conventional method by the detection of additional DNA polymorphisms.

A method according to the invention may also be useful for identifying high-volume co-dominant genetic markers or differences in complex or closely related genomes, and can easily be applied for genomic characterization of a variety of taxa.

### Description of the drawings

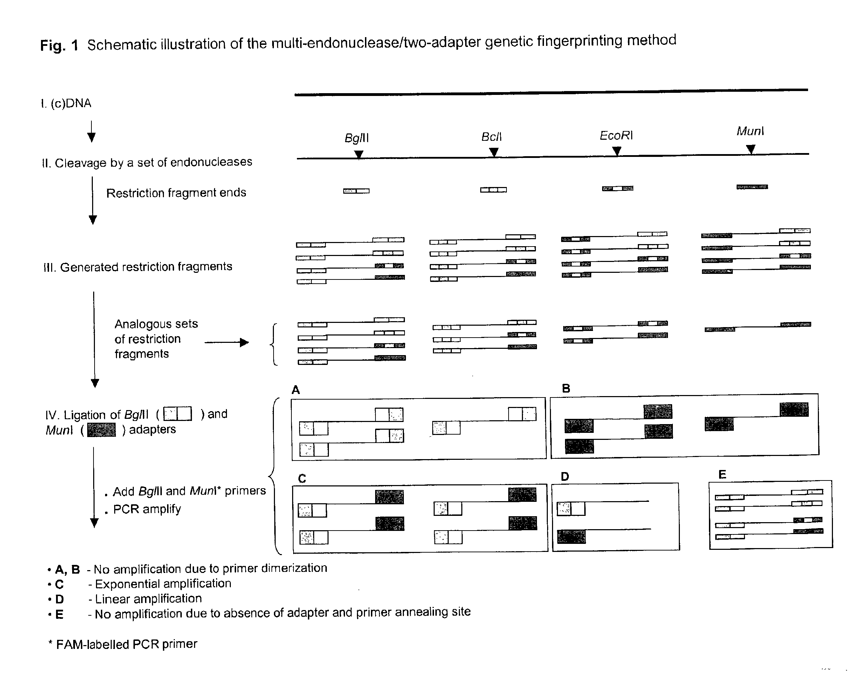

Figure 1 shows a schematic illustration of one embodiment of a method according to the present invention in which four endonucleases and two adapters are used.

In step I an amount of DNA is provided which is digested with four different restriction enzymes in step II. In this embodiment, a set of two pairs of restriction enzymes is used (*Bgl*II*-Bcl*I and *EcoR*I*-Mun*I). The digestion generates 16 different restriction products in step III, corresponding to 10 analogous sets of restriction fragments.

The solid lines represent restriction enzyme fragments of DNA. The boxes at the end of the various DNA fragments represent the respective restriction ends. Because, for each cognate pair of endonucleases, the cohesive ends created by one enzyme are compatible to the cohesive ends created by the other enzyme, the two adapters (*Bgl*II and *Mun*I) also ligate to cohesive ends created by the endonuclease enzymes *Bcl*I and *EcoR*I, respectively.

Following the ligation reaction, 5 different groups of products arise (step IV): sets carrying *Bgl*II or *Mun*I adapters at both ends (A, B); sets with *Bgl*II and *Mun*I adapters at the ends of each fragment (heterosite) (C); sets with *Bgl*II or *Mun*I adapter at only one end of fragment (D), and, sets that did not ligate to any adapter (E).

For selective PCR amplification and detection of fragments, *Mun*I primer is fluorescently labelled. Note that irrespective of which of the two cognate primers is labelled, the same subset of representation products, comprising the heterosite fragments, is exponentially amplified and detected (IV, C).

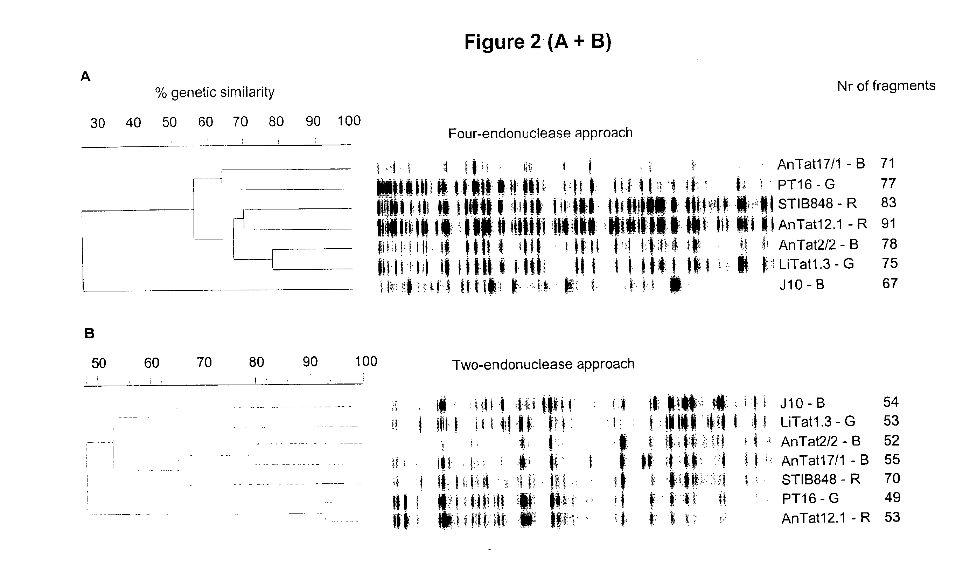

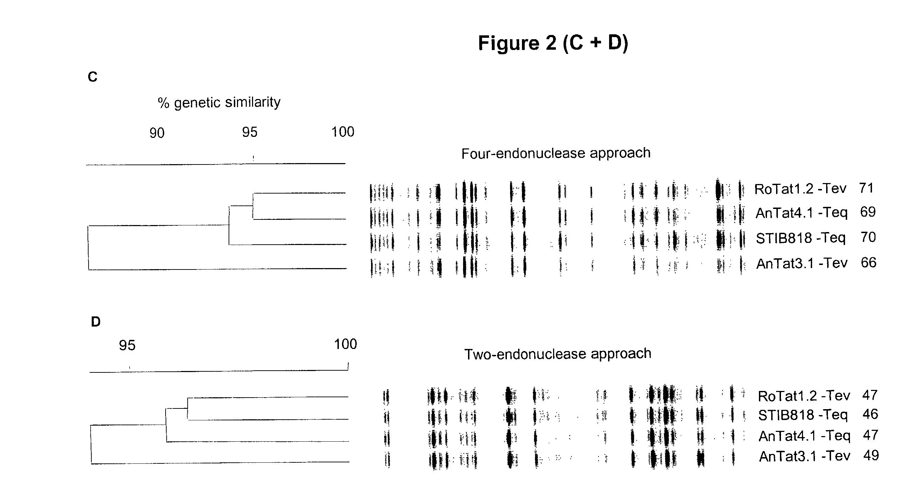

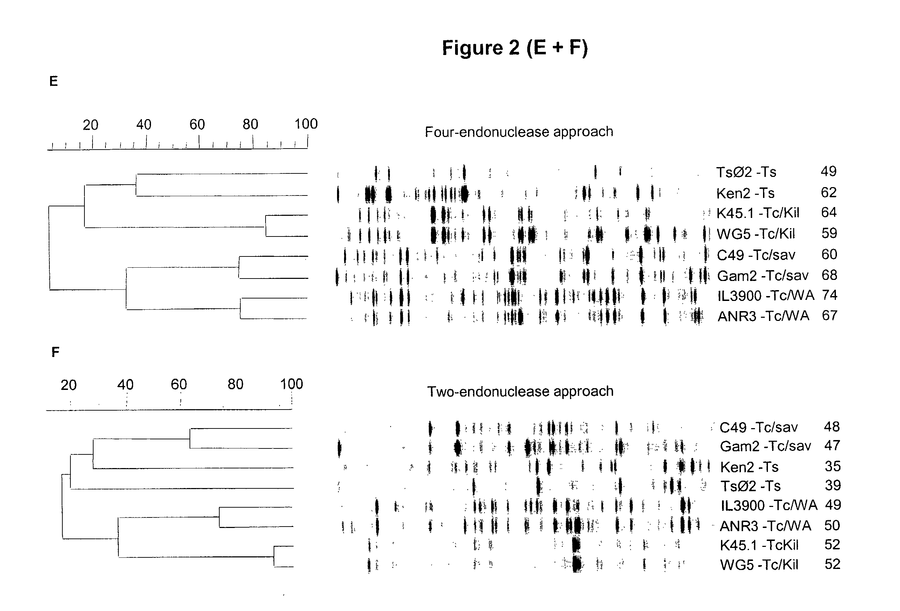

Figure 2 demonstrates the higher resolution power of the four endonuclease/two adapters approach.

Fingerprints resulting from intra- and inter-species analyses of trypanosome isolates demonstrate greater resolution of the four-endonuclease approach over the two-endonuclease approach. A, C and E represent dendograms from the samples analysed with the four endonuclease approach (using *Bgl*II-*Bcl*I/*EcoR*I*-Mun*I). These are directly compared with profiles derived from samples processed with the two endonucleases (*Bgl*II and *EcoR*I), viz B, D and F, respectively.

A pair of adapters and primers (*Bgl*II and *Mun*I) was used, respectively, to ligate and PCR amplify the resulting digestion products. The total number of bands from each tested sample is indicated.

### Detailed description of the invention

A method according to the invention may provide a robust and reproducible technique for generating high numbers of amplifiable fragments from DNA and may be used to detect high numbers of polymorphisms between e.g. genomes.

When, for example, a set of four 6 bp-cutting endonucleases, each of which recognizes a different DNA sequence, is used during a genomic DNA digestion according to a method of the present invention, this set will simultaneously access independent sites. Further, according to the present invention, the set of four 6 bp-cutting endonucleases are preferably chosen such that they form paired sets, wherein each paired set comprises two restriction enzymes that recognize different DNA signatures, but that create compatible cohesive ends. Such compatible cohesive ends being ligatable to a single adapter. Therefore, when using a set of four 6 bp-cutting endonucleases chosen such that they form paired sets according to the invention, only two adapters are required to produce amplifiable fragments.

When, for example, a combination of four restriction enzymes is used that consist of a paired set of the restriction enzymes *Bgl*II and *Bcl*I and another paired set of the restriction enzymes *EcoR*I and *Mun*I, only one pair of adapters, for example a pair consisting of a *Bgl*II adapter and a *Mun*I adapter (see Table 1), may be ligated to the various restriction fragments formed, thereby allowing the amplification of the restriction fragments by a single pair of cognate primers in PCR.

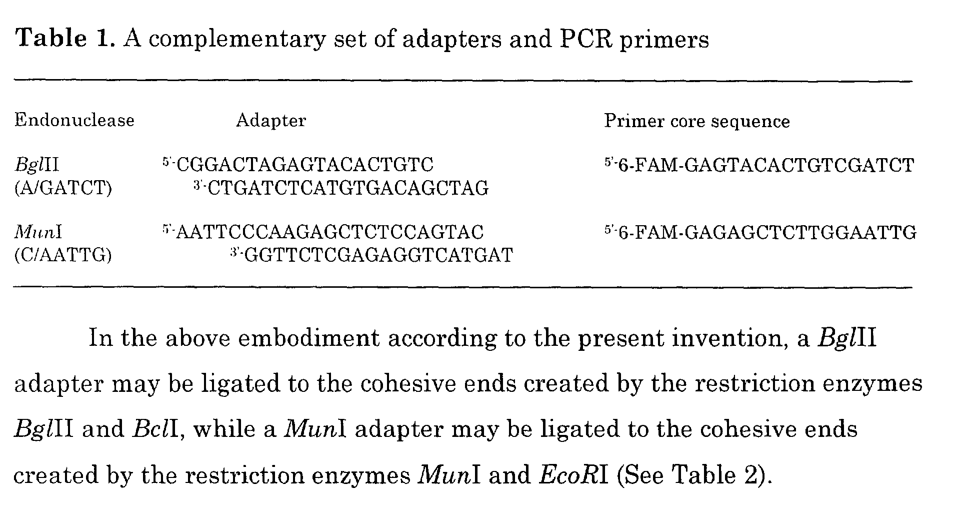

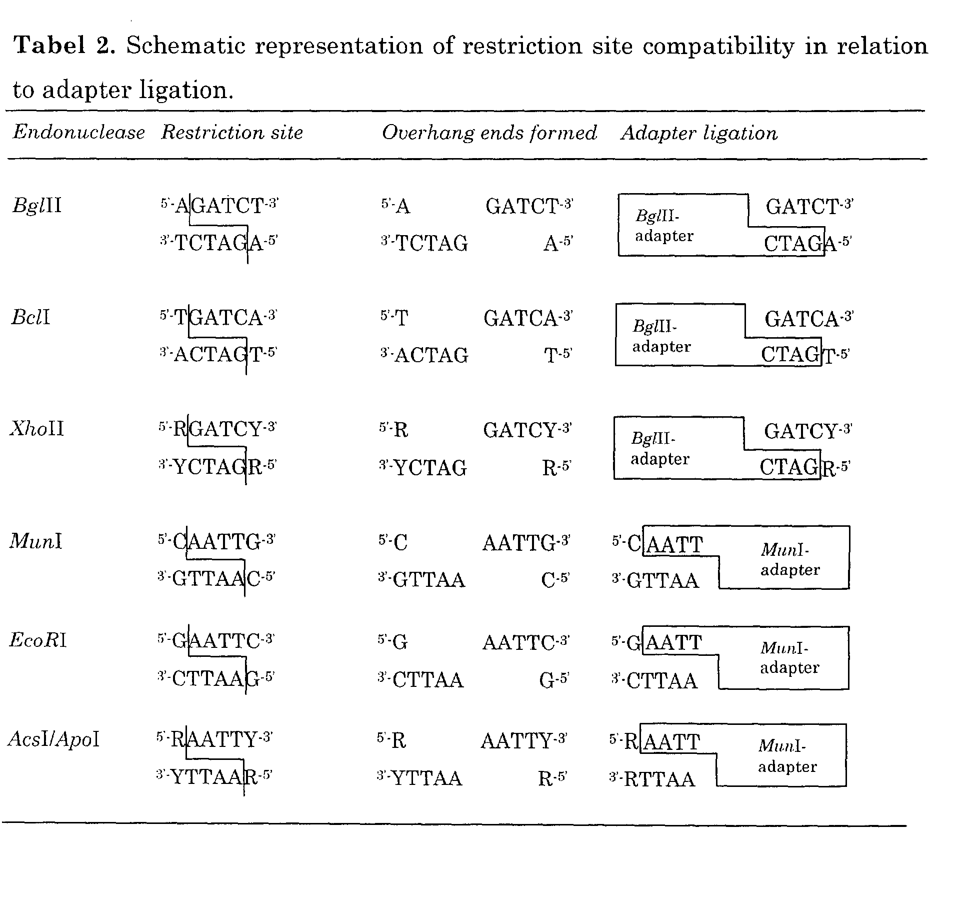

The possibility of using a short adapter ligation step allows for a substantial reduction in the amount of time required to prepare DNA fingerprints according to a method of the invention.

The above described embodiment, wherein the restriction enzymes *Bgl*II, *Bcl*I, *EcoR*I *and Mun*I are used in combination with adapter ligation to a *Bgl*II and a *Mun*I adapter, results in the generation of 5 groups of reaction products (Fig. 1, A - E). These consist of sets of fragments with (i) *Bgl*II or *Mun*I adapters at both ends (homosite fragments) (A, B), (ii) *Bgl*II and *Mun*I adapters at the opposing ends of each fragment (heterosite) (C), (iii) *Bgl*II or *Mun*I adapter at only one end of fragment (D) and, (iv) no adapter (E). A PCR reaction may then be used to obtain exponential amplification of the heterosite products (wherein different adapters are ligated to each end).

In contrast, DNA fragments with only one adapter undergo a linear amplification and are rapidly competed out in PCR, while the amplification of DNA fragments with the same adapter on each end (Fig. 1, A and B) is suppressed because the self-annealing of inverted repeat adapters inhibits the binding of the PCR primers.

The use of only one set of adapters and primers diminishes competition during PCR, thereby permitting stringent reaction conditions and resulting in robustness of the method.

It should be noted that the homosite fragments (i.e., fragments that have the same type of adapter at both ends) are also valuable for detecting polymorphisms, but they should be sufficiently enriched for in a reaction as their amplification would normally be suppressed as described. But since some homosite fragments may have originated from the annealing and ligation of adapters to restriction fragments that were created by the restriction site recognition and subsequent cleavage by two different endonucleases, such fragments may inherently contain polymorphisms of value to increasing the resolution of the typing method.

In the above described embodiment, in principle three different groups of PCR amplification products are generated, irrespective of which of the two selective primers is labeled (Fig. 1).

A method according to the present invention may be performed on a nucleic acid, such as a deoxyribonucleic acid or DNA, such as genomic DNA, mitochondrial DNA, plasmid DNA or cDNA or inserts or clones resulting thereof.

Said DNA may be derived from prokaryotic organisms such as bacteria or archaea and may also be derived from viruses. Also, DNA to be used in a method according to the invention may be derived from eukaryotic organisms such as eukaryotic micro-organisms like algae, fungi or yeast or eukaryotic organisms as plants, like higher plants, and animals, such as mammals. In fact, DNA originating from any source or from any organism may be used in a method according to the invention. The DNA may be obtained from a blood sample or other suitable biological sample.

The DNA may be isolated using any procedure known to the person skilled in the art for the extraction of DNA from a sample. The method by which the DNA is isolated or extracted is not essential in a method according to the invention. The DNA may optionally be purified before being used in a method of the invention using any procedure known to the person skilled in the art for the purification of DNA.

A method of the invention may comprise the unspecific or random fragmentation of DNA into fragments by using unspecific methods such as mechanical methods like sonication or French pressure cell methods, whereby the DNA is ruptured and fragmented. Also, physical methods such as heat treatment or chemical methods for random fragmentation of DNA such as acid hydrolysis may be employed in a method of the invention as well as combinations thereof.

Preferably, an unspecific or random fragmentation according to the invention generates DNA fragments of between 10 to 5000 nucleotides, more preferably, 20 to 500 nucleotides. Such random fragmentation may for example be achieved by sonication for a predetermined period of time using a standardized or optimized intensity of sonication.

The randomly fragmented DNA, comprising fragments with both blunt-ends and cohesive-ends, may optionally be treated such as to provide the DNA fragments with ends that are ligatable to an adapter. Such treatment may comprise the ligation of DNA fragments to linkers that are ligatable to an adapter. Alternatively such treatment may comprise a blunting procedure, wherein DNA fragments are treated such as to allow for the ligation of blunt-end adapters.

A method of the invention may also comprise the cleavage or cutting of DNA into fragments by using specific methods of fragmentation such as an S1 nuclease digestion, which may specifically digest A+T-rich DNA sequences of non-coding regions of a genome, thereby leaving the specific coding fragments of the DNA. Such specific methods of fragmentation of a starting DNA may be combined with other treatments such as heat treatment. For example, when heat-treating a starting DNA to the level of partial melting, such as melting at A+T-rich regions, such single stranded regions may then be treated by specific enzymes, thereby fragmenting the DNA in a specific manner. A specific method of fragmentation may also comprise the use of restriction endonucleases, or the use of compounds such as the camptethecin family of compounds or the enediyne antibiotics or other compounds, that preferentially bind to and cleave A+T-rich or G+C-rich DNA sequences or cause scission at minor grooves of the DNA, thereby leaving for example specific fragments or regions of the genome, that may then be analyzed independently.

Preferably, a specific fragmentation according to the invention generates DNA fragments of between 10 to 5000 nucleotides, more preferably, 20 to 500 nucleotides.

A method of the invention preferably comprises the fragmentation of the DNA in a specific manner through cutting of the starting DNA into fragments by specific combinations of restriction endonuclease enzymes. For this, a large number of restriction endonuclease enzymes is commercially available and suitable for use in a method of the invention.

The restriction endonuclease enzymes may comprise blunt-end cutting enzymes as well as enzymes that create overhanging or recessive ends ("sticky ends"), hereinafter called cohesive ends, of varying length, depending on the specificity of the respective endonuclease used.

Suitable endonucleases comprise such enzymes that can cut between 2 to about 13 bases in a double stranded DNA molecule, including such enzymes whose cutting sequence is located outside of its recognition sequence, but preferably cut between 4 to about 8 bases in a double stranded DNA, more preferably 4 to 7.

In a preferred embodiment according to the present invention, the DNA is cut with at least two endonucleases that create restriction ends that are compatible to ligation with a single adapter.

The term compatible as used herein for a nucleic acid fragment end, such as a restriction end, refers to the ability of this end to align and be joined by a ligation reaction to an adapter, when placed under conditions for ligation to occur, e.g. in the presence an agent for ligation and optionally nucleotides. Likewise, the term compatible as used herein for an adapter refers to the ability of this adapter to align and be joined by a ligation reaction to the end of a nucleic acid fragment end, such as a restriction fragment end, when placed under conditions for ligation to occur, e.g. in the presence an agent for ligation and optionally nucleotides. The term compatible as used herein for two different nucleic acid fragment ends, such as two restriction fragments created by the action of two different restriction endonuclease enzymes, refers to the ability of these ends to align and be joined by a ligation reaction to a single adapter.

As can be seen in Table 1, the adapter for the *Mun* I restriction site (as used in Example 3) is designed such that the target site for the restriction endonuclease is lost after ligation of the adapter to a *Mun* I-derived restriction fragment. In this case, a cytosine residue was inserted in the 3' direction of the annealing part of the overhang end of the adapter that is not base-paring with a corresponding base in the restriction fragment. Such an adapter design is useful when performing restriction and ligation procedures in one reaction, wherein the digestion of the newly ligated fragments is undesirable. In this case however, despite the presence of non-pairing bases, adapter ligation will readily occur between the adapter and a *Mun* I-derived restriction fragment.

Similarly, restriction sites created by different restriction endonucleases need not necessarily be identical to allow for a single adapter to be ligated to each of them. In fact, variations in restriction site sequence are tolerable as long as a single adapter can be ligated to such compatible restriction fragment ends. An example is the use of the restriction enzyme *Hga* I, with the recognition site GACGC(5/10), which may create a restriction end that is compatible to the restriction end created by the action of the enzyme *Mun I.* As used herein, restriction fragment ends are compatible when a single adapter can be ligated to both of them.

Besides the above mentioned at least two endonucleases that create compatible restriction ends, additional endonucleases may be used in a method according to the invention that are different from the two endonucleases that create compatible restriction ends. For such additional endonucleases, additional adapters may be provided in the case that amplification of the fragments produced from a DNA digestion with these enzymes is required.

In a preferred embodiment according to the present invention, a second set of at least two endonucleases that creates compatible ends is provided. Such a second set may be provided simultaneously or successively to a first set of at least two endonucleases that create compatible cohesive ends and may also comprise such enzymes that can cut between 2 to about 13 bases in a double stranded DNA molecule but preferably cut between 4 to about 8 bases in a double stranded DNA, more preferably 4 to 7. Such an embodiment comprises the provision of a first set and a second set of restriction endonuclease enzymes that produce compatible cohesive ends within each set. As a result, only two cognate adapters, one compatible to the first set and one compatible to the second set of restriction endonuclease enzymes, are required. Alternatively, the first and second set of restriction endonuclease enzymes may each comprise a pair of restriction endonuclease enzymes, each pair consisting of two restriction endonuclease enzymes capable of cutting a starting nucleic acid in a plurality of restriction fragments with compatible cohesive ends.

The invention provides a method wherein a specific fragmentation may comprise the use of at least four different restriction endonuclease enzymes, of which enzymes at least two produce restriction fragments with ends that are compatible to a first adapter as well a method wherein said at least four different restriction endonuclease enzymes comprise at least a further two enzymes that produce restriction fragments with ends that are compatible to a second adapter.

Very suitable combinations of endonuclease enzymes that can be used in a method of the invention are e.g. those provided in Table 3, which enzymes create restriction ends that are essentially identical. This table describes for a number of endonuclease enzymes the corresponding enzymes that create compatible cohesive ends and that can be selected to form a paired set of restriction enzymes according to the invention.

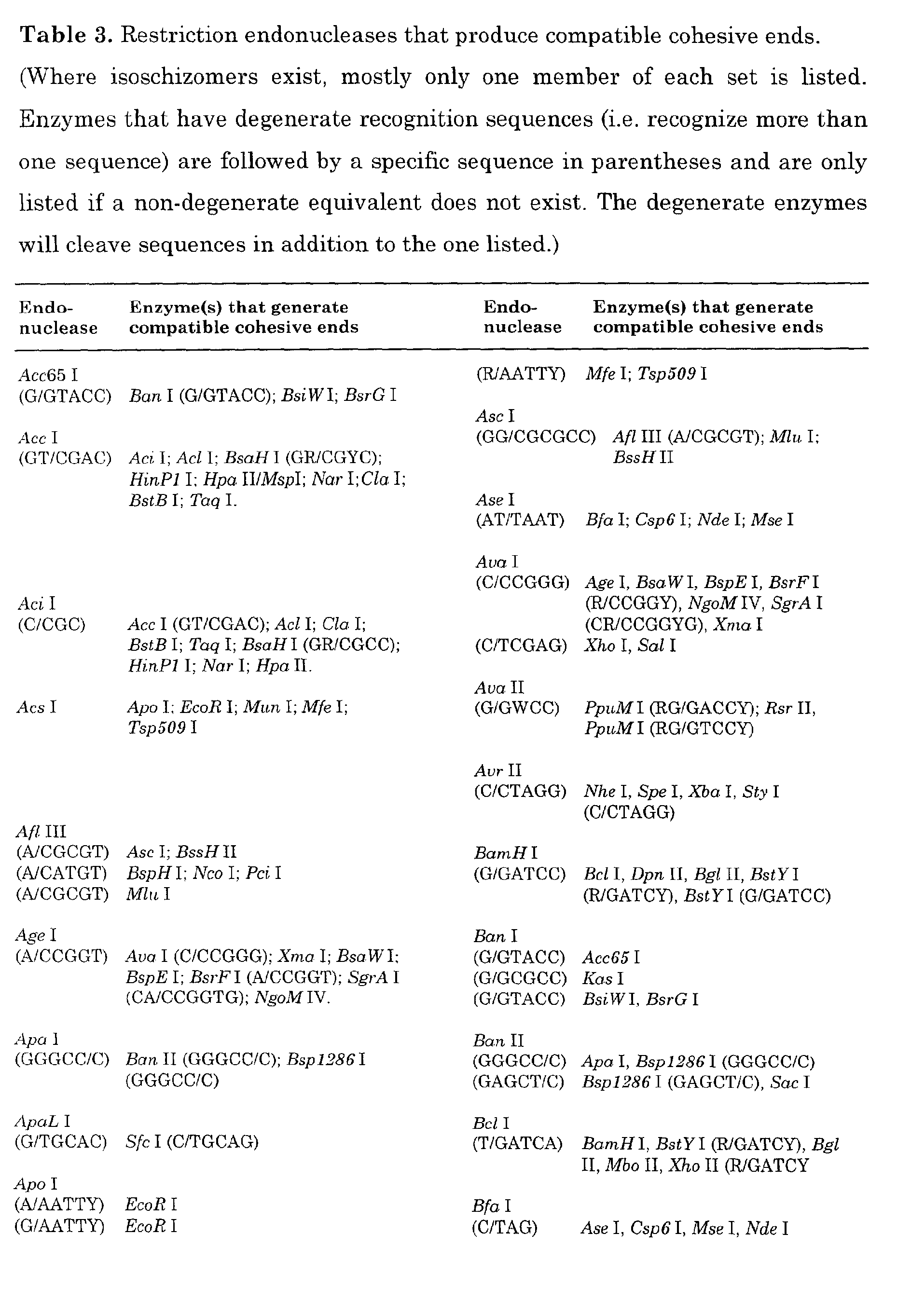

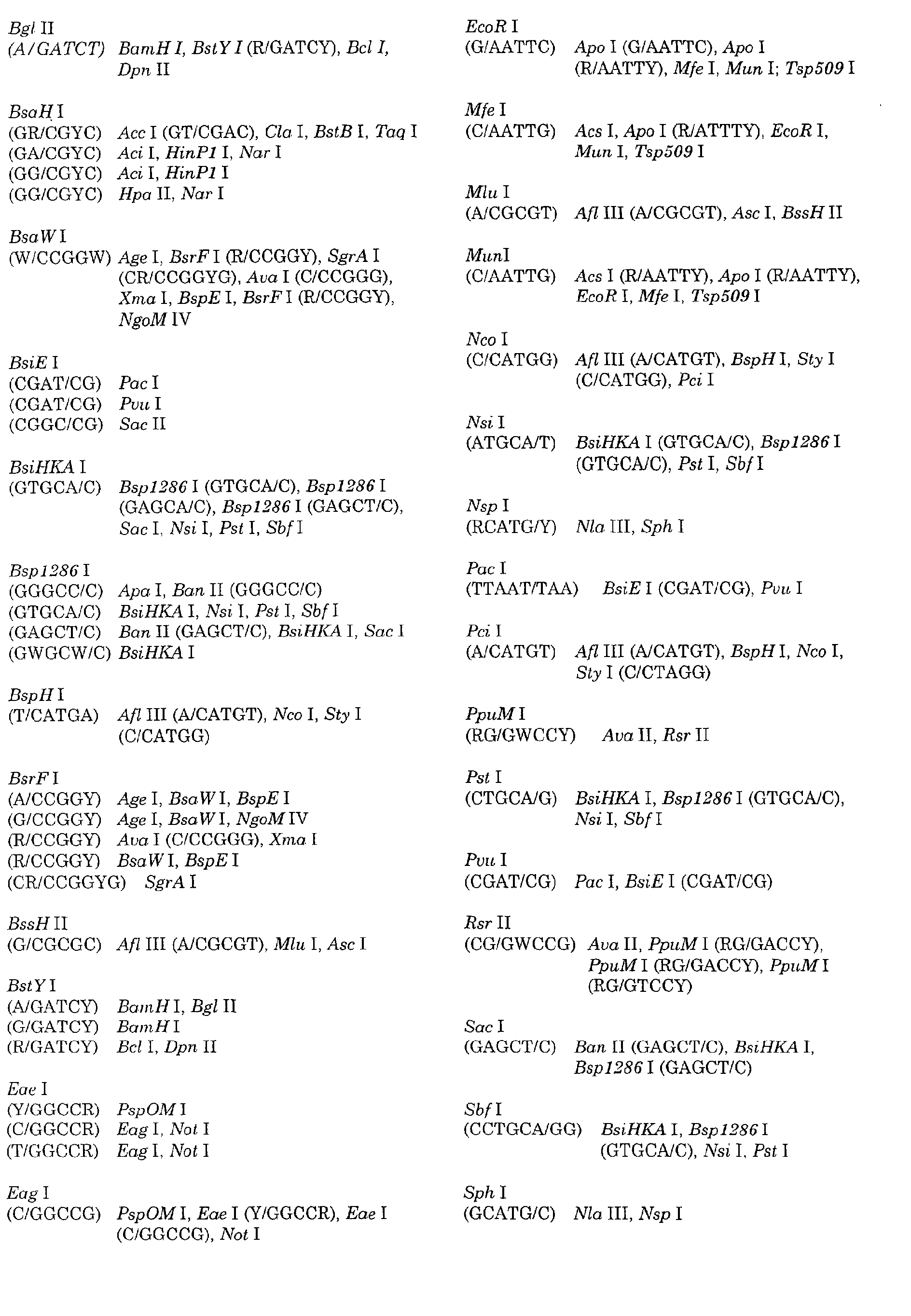

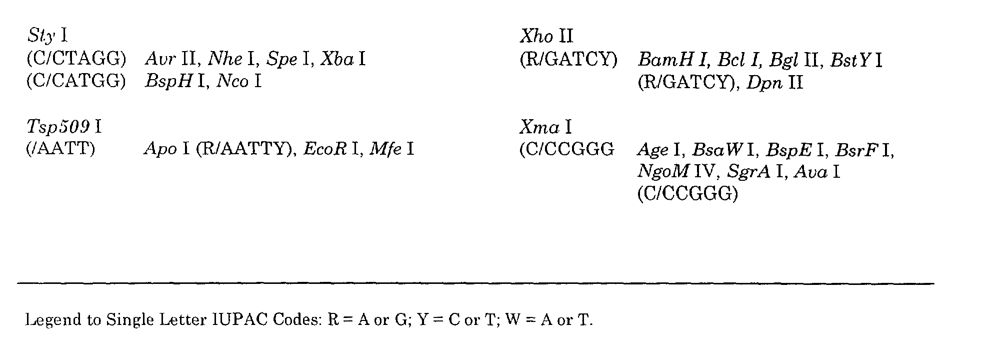

More preferably, the digestion of DNA according to a method of the invention comprises the use of at least two different restriction endonuclease enzymes that are selected on the basis of Table 3.

In a most preferred method according to the invention, paired sets of different restriction endonuclease enzymes are chosen from the group consisting of (i) *Bgl*II, *Bcl*I, *EcoR*I and *Mun*I; (ii) *Bgl*II, *Bcl*I, *Acs*I and *Mun*I; (iii) *BclI, Xho*II, *Acs*I and *Mun*I; and (iv) *Bgl*II, *Xho*II, *EcoR*I and *Acs*I.

The digestion of the DNA with a selection of restriction endonucleases may suitably occur in a reaction mixture comprising an aqueous solution that provides a buffered environment optimized for the activity of the respective restriction endonuclease. Such buffers are usually provided by the manufacturer along with the restriction endonuclease.

A suitable amount of DNA in a reaction mixture for performing a restriction endonuclease digestion of DNA according to the invention would be in a range of between about 1 to about 1000 ng, preferable between about 10 to about 250 ng, more preferably between 50 and 150 ng per reaction mixture.

A suitable amount of restriction endonuclease enzyme in such a reaction mixture is generally between about 0,1 U and about 500 U of enzyme, for each restriction endonuclease enzyme used. Preferably an amount of between about 1 U and about 100 U is used, more preferably between about 5 U and about 20 U.

A restriction endonuclease digestion of DNA according to the invention may be performed for a period of between 10 min and between 24 hours, depending on the amount of enzyme used and the reaction temperature. Usually, optimal temperatures for the endonuclease activity of the enzyme are used with a preferred reaction period of about 1-10 hours. It is preferred that the reaction proceeds until digestion is essentially complete.

A restriction endonuclease digestion reaction of DNA according to the invention may be performed in one single reaction with all restriction endonuclease enzymes present. It is also possible to perform the restriction endonuclease digestion reaction of DNA in two successive reactions.

Such an embodiment is not essential, but is preferred when using two sets of restriction endonuclease enzymes that produce compatible cohesive ends or a set of two pairs of restriction endonuclease enzymes that produce compatible cohesive ends. In such a latter case, two successive double digestion reactions are preferred. Between such successive digestion reactions, the DNA may optionally be precipitated, for example by using a precipitating agent such as 2-propanol or ethanol.

Upon completion of the restriction endonuclease digestion reaction, the DNA fragments may be precipitated, for example by using a precipitating agent such as 2-propanol or ethanol, and may be reconstituted in distilled water prior to use in a ligation reaction or and stored for later use in such a reaction.

Suitable adapters for use in embodiments of the invention comprise double stranded DNA adapters with ends that are compatible to ends of the DNA fragments obtained in the fragmentation of the starting DNA, such as restriction fragments in case that restriction endonucleases are used for fragmentation of DNA.

When using blunt-end adapters according to a method of the invention, DNA fragments with blunt-ends may suitably be created from DNA fragments with cohesive-ends by using ss-DNA digesting enzymes such as Exonuclease I, Mung Bean nuclease, S1 nuclease or exonuclease VII. Alternatively, a 5' overhanging or recessive end of a nucleic acid fragment may be filled-in by using DNA polymerases such as Klenow (fragment of *E. coli* DNA polymerase I), T4 DNA polymerase, or Pfu polymerase which would also remove 3' overhanging or recessive ends by the inherent 3' to 5' exonuclease activity, also leaving a blunt end on the nucleic acid fragment.

A method of the invention may suitably comprise the use of adapters that are blunt at one end and cohesive at the other end to allow for ligation of that adapter to the blunt-end nucleic acid fragment in a specific orientation.

As described, the term compatible as used herein refers to the ability of an adapter to align and be joined by a ligation reaction to a nucleic acid fragment, either produced by an unspecific or by a specific process for fragmentation, when placed under conditions for ligation to occur, e.g. in the presence an agent for ligation and optionally nucleotides, i.e. the adapter and the nucleic acid fragment are said to be compatible.

In the case that the nucleic acid fragment, such as a restriction fragment, and the adapter both comprise cohesive ends, the aligning will involve at least partial annealing of complementary overhanging ends. This means that the overhanging end of the adapter must be sufficiently complementary to hybridize with the respective overhanging end of the nucleic acid fragment in order to be ligated. Therefore, the sequence of the overhanging end of the adapter need not reflect the exact sequence of the respective overhanging end of the nucleic acid fragment, but may differ slightly in sequence or length.

In the present invention, an adapter and a nucleic acid fragment have compatible cohesive ends when these cohesive ends are substantially complementary. In fact, when a DNA fragmentation procedure comprises the use of at least two different restriction endonuclease enzymes that produce cohesive ends, it is sufficient for these cohesive ends to be substantially similar in order to be compatible to a single adapter.

It is a preferred embodiment of the present invention to use at least two adapters in a ligation reaction of the present invention. Said at least two adapters preferably comprise a first and a second adapter, one of which is ligatable to the cohesive ends formed by at least two different restriction endonuclease enzymes used in the DNA digestion reaction.

In an even more preferred embodiment, at least two adapters are used of which a first adapter is ligatable to the cohesive ends formed by a first set of at least two restriction enzymes capable of creating compatible cohesive ends, and a second adapter is ligatable to the cohesive ends formed by a second set of at least two restriction enzymes capable of creating compatible cohesive ends.

Although more than two adapters may be used in embodiments of the present invention, the use of two adapters with a corresponding cognate amplification primer pair constitutes a most preferred embodiment of the present invention to ensure robustness of the method.

When using blunt-end adapters according to a method of the invention, the nucleic acid fragments may suitably be created by blunt-end cutting enzymes. Alternatively, cohesive ends, generated by cohesive ends-cutting endonucleases may be facilitated with blunt end by ss-DNA digesting enzymes such as Exonuclease I, Mung Bean nuclease, S1 nuclease or exonuclease VII. Alternatively, a 5' overhanging or recessive end of a nucleic acid fragment may be filled-in by using DNA polymerases such as Klenow (fragment of *E. coli* DNA polymerase I), T4 DNA polymerase, or Pfu polymerase which would also remove 3' overhanging or recessive ends by the inherent 3' to 5' exonuclease activity, also leaving a blunt end.

When using restriction enzymes that generate blunt end restriction fragments, a method of the invention may comprise the use of adapters that are blunt at one end and cohesive at the other end to allow for ligation of that adapter to the blunt-end restriction fragment in a specific orientation. Such adapters that are blunt at one end and cohesive on the other end may be generated by the action of restriction endonuclease on double-stranded oligodeoxy ribonucleotides or oligonucleotides. Alternatively such adapters may be generated by the annealing of two specifically designed ss-oligodeoxy ribonucleotides that differ in length and that anneal such as to produce a ds-oligodeoxy ribonucleotide having one blunt end and one overhanging or recessive end.

Suitable adapters for use in a method according to the present invention may have a G+C content of about 50%, a length of about 3 to 50 bases, preferably 6-30 bases, more preferably 6-20 bases in case of a blunt-end adapter and 18-30 bases in case of a cohesive-end adapter and a melting temperature Tᵢₙ of about 40-65°C, preferably 55-60°C.

Ligation of the nucleic acid fragments produced in the fragmentation procedure, such as a restriction endonuclease digestion reaction, to adapters may suitably occur in a ligase reaction mixture comprising an aqueous buffer (for example a 330 mM Tris-HCl buffer at pH 7.5) and such compounds as MgCl₂, dithiothreitol (DTT), ATP and optionally compounds such as polyethyleneglycol (PEG). The amounts of these reagents are not essential to the invention but allow for optimal ligation reaction conditions. Suitable buffers are mostly provided by the commercial supplier of the ligase enzyme and usually comprise about 10-50 mM of MgCl₂, about 10 mM of dithiothreitol (DTT) and 0.5-10 mM of ATP.

Various ligase enzymes may be used in the ligase reaction according to the present invention. Suitable ligases are for example T4 DNA ligase or T7 DNA ligase. Again, the amount of ligase used in a ligase reaction in accordance with embodiments of the present invention is not essential. Suitable amounts are in the order of between 5 U to about 1000 U, preferably about 10 U to about 500 U in the case of T4 DNA ligase.

The ligase reaction mixture further comprises at least two adapters in an amount of between 1 to about 100 pM each, preferably about 10 to about 50 pM each, depending on the amount of nucleic acid fragments derived from the starting nucleic acid, or on the cutting frequency of restriction enzymes used in a specific fragmentation procedure with restriction enzymes. Usually the adapters are present in excess over the obtainable nucleic acid fragments. The ligase reaction mixture further comprises the reconstituted nucleic acid fragments such as produced in a restriction endonuclease digestion of o staring nucleic acid as described hereinabove in an amount of about 1 to about 1000 ng, preferable between about 10 to about 250 ng, more preferably between 50 and 150 ng per reaction mixture.

A ligase reaction according to the invention may be performed for a period of between 10 min and between 24 hours, depending on the amount of enzyme used and the reaction temperature. Usually, a temperature of about 25°C is used with a preferred reaction period of about 0.1-10 hours, more preferably about 2 hours. It is preferred that the reaction proceeds until ligation is essentially complete.

A method according to the invention further comprises the amplification of adapter-ligated nucleic acid fragments. Such an amplification reaction may comprise any suitable DNA amplification reaction known to the art, such as for example by PCR (Mullis, 1987; U.S. Pat. No. 4,683,202), optionally in a nested, a multiplex or an asymmetric setup, or by a ligase chain reaction (Barany, 1991), a self sustained sequence replication reaction (Guatelli *et al*., 1990), a transcriptional amplification system (Kwoh *et al*., 1989), a Q-Beta Replicase reaction (Kramer and Lizardi, 1990), a rolling circle amplification reaction (Lizardi *et al*., 1998), a boomerang DNA amplification reaction (BDA) (U.S. Pat. No. 5,470,724) or any other nucleic acid amplification method.

A preferred amplification reaction for use in a method according to the invention comprises a PCR amplification reaction by using Taq polymerase. Such methods are routinely employed in the art and make use of primers.

The term "primer" as used herein refers to an oligonucleotide, whether occurring naturally or produced synthetically, which is capable of acting as a point of initiation of synthesis when placed under conditions in which synthesis of primer extension product, which is complementary to a nucleic acid strand is induced, i.e., in the presence of nucleotides and an agent for polymerization such as DNA polymerase and at suitable a temperature and pH. The primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primer will depend on many factors, including temperature and source of the primer.

The primers herein are selected to be "substantially" complementary to the different strands of each specific sequence to be amplified. This means that the primers must be sufficiently complementary to hybridize with their respective strands. Therefore, the primer sequence need not reflect the exact sequence of the template. In the present invention, the primers are substantially complementary to the adapters.

Prior to a selective amplification reaction, the nucleic acid fragments ligated to the adapters may optionally be subjected to a pre-selective amplification reaction. Such a reaction is meant to minimize artifacts and enrich for the amplification of heterosite nucleic acid fragments.

The PCR amplification primers preferably bind selectively to the complementary sequences of adapters ligated to the nucleic acid fragments. Stringent PCR conditions may be required in some applications wherein a method according to the invention is required. The stringency requirements may be most determinative to the nature of the PCR primers, i.e. their length expressed as the number of nucleotides, the most suitable annealing site on the adapter, their G+C content, their annealing temperature, etc. All these parameters may be optimized by those skilled in the art and are not essential to the present invention. A suitable length of the primers is between 8-40 nucleotides.

It is preferred that the primer anneals by perfect base pairing with the DNA sequence of one of the DNA strands at the end of the adapter-ligated nucleic acid fragment.

In order to amplify a subset of the adapter-ligated nucleic acid fragments, at least one primer of the pair of primers used in the amplification reaction may comprise from 1-5 selective nucleotides, preferably from 1-3 selective nucleotides at its 3' end that . The expression "selective" herein designates the presence of nucleotides that base-pair with unknown nucleotides in the nucleic acid fragment such as those that flank the target site of the restriction endonuclease in the 3'-5' direction of the adapter-ligated end of a restriction fragment. This measure may provide optimized discrimination by preventing the annealing of the PCR primer in the case of non-complementarity of the region such as is the case when SNP's or other nucleotide polymorphism are present close to the restriction site.

One or both of the PCR primers may be labeled to facilitate the detection of the amplification products. Preferably one primer is labeled. Suitable labels comprise radioactive labels, chromogenic labels, luminescent labels, phosphorescent labels, fluorescent labels such as FAM, TET, TAM, ROX, SYBR Green, Cy3, Alexa, or Texas Red, all of which are well known in the art, or other labels for detecting such tagged fragments.

Upon their amplification, the nucleic acid fragments may be detected such as to form a fingerprint of the source or organism from which the nucleic acid was extracted. A variety of different methods may be employed ranging from separation of the fragments based on size or denaturing profile on, for example, an electrophoresis gel, a capillary gel, a metaphor gel, or any other gel matrix, or by chromatography, or by an array of allelic and non-allelic markers on a DNA array.

A fingerprint is thus understood to comprise a profile of nucleic acid fragments such as a 1-dimensional banding pattern or a 2-dimensional separation pattern or an array of positive and negative spots on a support substrate.

When using sequencing gels and phospho-imaging or autoradiographic means for detection of bands, the amplification primers may accordingly be labeled to allow for such detection. A preferred embodiment comprises the use of denaturing (polyacrylamide) sequencing gels such as used in automated DNA sequencers with fluorimetric detection which will coincide with the use of at least one fluorescently labeled amplification primer, such as a FAM-labeled amplification primer and fluorescent detection.

In embodiments wherein a detection results in the formation of a discrete banding pattern wherein each band corresponds to a discrete nucleic acid fragment, the banding pattern may be scanned and analyzed by using analysis software dedicated to such tasks, such software being com commercially available, resulting in a quantifiable fingerprint of the nucleic acid analyzed.

By comparing different DNA fingerprints prepared by a method according to the invention as set forth hereinabove, a method for detecting e.g. sequence polymorphisms between one or more genomes is provided. Further, various nucleic acid fingerprints may be comparatively analyzed for the presence and or absence of specific nucleic acid fragments.

A method for detecting DNA sequence polymorphisms according to the present invention provides the high resolution power that is required for analyzing diversity, establishing classification, or identifying taxa based on genomes from e.g. lower level taxa such as between species, subspecies, strains, variatas, forms, cultivars or sub-populations.
Individual nucleic acid fingerprints and comparative analysis data resulting from a method according to the present invention may be stored into a computer database. In conjunction with suitable software, such a database may further be used for taxonomic or classification purpose. Also, identification of unknown organisms may be achieved by using a method for the preparation of a nucleic acid fingerprint according to the invention in combination with a database comprising a plurality, or essentially at least one, of such fingerprints. Such a database may comprise fingerprints prepared earlier from related organisms by that same method. Such a method for identification of unknown organisms may comprise comparing said fingerprint with at least one nucleic acid fingerprint prepared from at least one known organism by that same method and establishing the identity of said unknown organism on the basis of similarity of said nucleic acid fingerprint from said unknown organism with said at least one nucleic acid fingerprint from said at least one known organism.

Use may be made of a method according to the invention for detecting sequence polymorphisms between e.g. one or more genomes for the preparation of genetic markers. Such markers may suitably comprise SNP markers, multi-locus markers, single locus markers, dominant markers, diagnostic markers for assessing genetic differences or relatedness among individuals, populations, species, subspecies, strains, variatas, forms, cultivars, or subpopulations, phenotypic markers derived from, e.g. pedigree analysis, clonal markers to follow, e.g. recombinant gene flow and dispersal, out-crossing, introgression and hybridization, or reference markers for genetic mapping studies. Such markers may be used as molecular signatures linked to specific phenotypes and may be used to detect DNA polymorphisms associated with specific phenotypic characteristics.

It is an embodiment of the present invention to provide a kit for preparing a DNA fingerprint according to the present invention, which kit may comprise a set of restriction endonuclease enzymes and buffers for use in a method according to the present invention, a set of double stranded DNA adapters, DNA ligase enzymes and buffers, a set of cognate primers for initiation of DNA amplification optionally labeled with a suitably detectable label, DNA polymerase enzymes and buffers, reference DNA for use in a fragmentation or a restriction digestion as a sizing standard and/or data analysis software essential to performing a method according to the invention.

The invention will now be illustrated by means of the following examples, which are in no way intended to limit the scope of the present invention.

### Example 1

### Theoretical evaluation of the multiplex-endonuclease, 2-adapter genetic fingerprinting approach.

When a 6 bp-cutting endonuclease is used to digest genomic DNA, then, on average, a restriction site occurs every 4096 (4⁶) bp, assuming a random distribution of the four bases in the DNA.

When a set of four different 6 bp-cutting endonucleases, that simultaneously access independent sites, are used to digest the same genomic DNA, the cleavage site of each restriction enzyme would be expected to occur approximately every 1024 bp, versus approximately every 2048 bp in case that only two 6-bp cutting enzymes are used. In practice, this will not be the case as will be discussed hereinbelow.

Assuming a random distribution of four different 6-bp-recognizing ('rare cutting') enzymes (hypothetically *A, B, C* and *D* combination), the probability, P of their respective digestion frequency in the genome is (P_{*AB*} + P_{*CD*}). Further assuming that the restriction enzyme cleavage sites are independent of one another and do not share nucleotides at similar positions, P is 4⁴ (4² + 4²) + 4⁴ (4² + 4²) = 4⁵.

Taking, for example, the set of four 'rare cutter' enzymes used in Example 3 as described hereinbelow (*Bgl*II*-Bcl*I/*EcoR*I*-Mun*I), this set would generate sets of fragments with 16 possible restriction ends out of which 10 different sets are potentially amplifiable (Fig. 1). The restriction enzymes were chosen such that for each pair, the cohesive ends created by one are compatible to the overhanging or recessive ends created by the other restriction enzyme. On this basis, only one pair of adapters, *Bgl*II and *Mun*I (Table 2) was ligated to allow the amplification of the fragments using a pair of cognate primers in PCR. In this approach, *Bgl*II adapter also ligated to the overhanging or recessive ends created by BclI or *Xho*II, while *Mun*I adapter also ligated to *EcoR*I, *Acs*I or *Apo*I sites. The short adapter ligation step ensured a substantial reduction in the amount of time required for preparing the DNA fingerprints.

With the *Bgl*II, *Bcl*I, *EcoR*I *and Mun*I combination, following adapter ligation reaction, 5 groups of products result (Fig. 1, A - E). These consist of sets of fragments with (i) *Bgl*II or *Mun*I adapters at both ends (A, B), (ii) *Bgl*II and *Mun*I adapters at the ends of each fragment (heterosite) (C), (iii) *Bgl*II or *Mun*I adapter at only one end of fragment (D) and, (iv) no adapter (E). The PCR was then used to obtain exponential amplification of the heterosite products (with different adapters at each end). In contrast, DNA fragments with only one adapter undergo linear amplification and are rapidly competed out in PCR. The amplification of DNA with the same adapter at each end (Fig. 1, A and B) is suppressed because self-annealing of inverted repeat adapters inhibits binding of PCR primers.

The use of only one set of adapters and primers diminishes competition during PCR, permitting robust and stringent reaction conditions. Repeat of the experiments with samples derived at two separate times from the 'test' isolates gave identical AFLP profiles, with the same bands and same polymorphisms which indicates high reproducibility of the method.

### Example 2

### Computer predictive modelling

For computer predictive modelling, a Monte Carlo simulation program (in QuickBasic language) was used to calculate an estimate of the occurrence of the recognition enzyme sequences. The program randomly generated a genome of adequate large size, to calculate the frequency of the recognition sequences of a set of restriction enzymes, with diverse combinations and permutations. Increasing the size of the genome and the number of repetitions was used to enhance the precision of the estimate. Also the program is able to analyse known genome sequences. With this system it was possible to predict the suitability of various restriction enzyme combinations in a method according to the invention.

### Example 3

### Four-endonuclease, two-adapter genetic fingerprinting

Sets of four endonucleases in combination two adapters were used to evaluate the value of the multiplex-endonuclease, two-adapter method as a useful tool for high-resolution DNA fingerprinting.

To identify the best set of endonucleases, we first evaluated the approach using seven 'test' *Trypanosoma brucei* isolates belonging to the three subspecies of the parasite - *T. b. brucei, T. b. gambiense* and *T. b. rhodesiense* (Table 4). Genomic DNA, isolated essentially as previously described (Heath, 1997), was digested using restriction enzyme combinations: (i) *Bgl*II, *Bcl*I, *EcoR*I *and Mun*I; (ii) *Bgl*II, *Bcl*I, *Acs*I and *Mun*I; (iii) *Bcl*I, *Xho*II, *Acs*I and *Mun*I, and; (iv) *Bgl*II, *Xho*II*, EcoR*I and *Acs*I, respectively. All enzymes were purchased from Roche Molecular Biochemicals (Almere, The Netherlands) or Westburg (Leusden, The Netherlands).

**Table 4**

| Trypanosome isolates used | | |
|---|---|---|
| Isolate | Source | Year of isolation |
| *Trypanosoma brucei brucei* | | |
| AnTat2/2* | Tsetse fly (*Glossina morsitans*); Nigeria | 1970 |
| AnTat17/1* | Sheep; Democratic Rep. of Congo | 1978 |
| J10* | Hyena; Zambia | 1973 |

| *T. brucei gambiense* | | |
|---|---|---|
| LiTat1.3* | Human; Cote de Ivoire | 1952 |
| PT16* | Human; Cote de Ivoire | 1992 |

| *T. brueci rhodesiense* | | |
|---|---|---|
| STIB848* | Human; Uganda | 1990 |
| AnTat12.1* ⁺ | Human; Uganda | 1971 |

| *T. evansi* | | |
|---|---|---|
| RoTat1.2 | Water buffalo; Indonesia | 1982 |
| AnTat3.1 | Capybara; South America | 1969 |

| *T. equiperdum* | | |
|---|---|---|
| STIB818 | Horse; China | 1979 |
| AnTat4.1 | Unknown; South America | N/A |

| *T. corigolense* | | |
|---|---|---|
| C49 (savannah) | Cow, Kenya | 1966 |
| Gam2 (savannah) | Cow; The Gambia | 1977 |
| IL3900 (riverine/forest) | Dog; Burkina Faso | 1980 |
| ANR3 (riverine/forest) | Fly; The Gambia | 1988 |
| K45.1 (kilifi) | Cow; Kenya | 1982 |
| WG5 (kilifi) | Goat; Kenya | 1980 |

| *T. sliniae* | | |
|---|---|---|
| Ken2 | Fly; The Gambia | 1988 |
| TsØ2 | Bushbuck; Kenya | N/A |

| | | |
|---|---|---|
| * 'Test' samples; ⁺ Adapted sensitive to normal human serum; N/A - data not available | | |

Since complete digestion of DNA is an important step in the present technique, 100 ng genomic DNA was digested for 4 h with 10 U of each endonuclease, essentially in two successive double digestion reactions, with an intermediate 2-propanol precipitation step.

The digests were precipitated and reconstituted in 10 µl distilled water. Ten µl of a buffer containing 660 mM Tris HCl, 50 mM MgCl₂, 10 mM dithiothreitol, 10mM ATP, pH7.5, and 20 pM each of *Bgl*II and *Mu*nI adapters (Table 1) were added. One µl (400U) of T4 DNA ligase (New England Biolabs) was added and the mixture was incubated for 2 h at 25°C.

A pre-selective amplification was performed in a total volume of 20 µl containing 1 U of Taq polymerase (Roche Molecular Biochemicals, Almere, The Netherlands), 4 µl of 1:1-diluted ligation product, 2 µl of 10X PCR buffer (100mM Tris HCl pH 9.0, 50 mM KCl, 1% triton X-100, 0.1% w/v gelatin), 2.5 mM MgCl2, 200 µM of each dNTP and 5 pM of each *Bgl*II and *Mun*I primers. The reaction mixture was incubated for 2 min at 95°C, and subjected to 20 cycles of PCR (30 s at 95°C, 30 s at 56°C and 2 min at 72°C).

Four µl volumes of 1:20-diluted pre-selective products were used as template for a selective amplification reaction. The PCR program was essentially the same as for pre-selective amplification, except that the cycling step was followed by 30-min incubation at 60°C.

The final products were diluted 1:1 with TE. To a 1 µl volume of the diluted product a Genescan-500 internal lane standard (PE Applied Biosystems) was added and the mixture was resolved in a 7.3% denaturing sequencing gel using a model ABI 373A automated DNA sequencer. Gels were routinely prepared by using ABI protocols and subjected to electrophoresis for 5 h.

To assess the reproducibility of the approach, two sets of genomic DNA were isolated at separate occasions from the 'test' isolates, and processed according to the above mentioned protocol.

For data analysis, gel patterns were collected with GeneScan software (PE Applied Biosystems) and sample files were transferred to the GelCompar v4.1 software (Applied Maths, Kortrijk, Belgium). Band positions were inferred from the signal positions in the sample files, and used to establish the total number of bands in each lane.

By computer-assisted analysis, sample files were directly scored for presence/absence of signal peaks in the four-endonuclease or conventional approach. Gels were normalized by using the internal standard that was added to each lane. The Pearson product-moment correlation coefficient (Pearson, 1926, *Biometrika* **18**, 105-117) was used to create a similarity matrix. Clustering of the patterns was performed with the unweighed pair group method using average linkage (UPGMA) (Vauterin & Vauterin, 1992, *European Microbiol. J.* 1, 37-41).

The approach evaluated using four-endonucleases in combination with two adapters was found to be a robust and reproducible technique capable of detecting additional amplifiable fragments to increase the number of detectable polymorphisms between genomes.

### Example 4

### Multi-endonuclease restriction pattern approach.

As an embodiment of the present invention we describe herein an approach wherein the simultaneous use of four restriction endonucleases is able to provide greater resolution and produce more identifiable polymorphic fragments, in combination with one pair of adapters/primers for stringent PCR.

The method takes advantage of the fact that some endonucleases create cohesive ends that are compatible to the overhanging or recessive ends created by other endonucleases. On this basis, we genotyped 19 trypanosome isolates (Table 4) to the subspecies level, and demonstrated the greater fingerprinting power of this approach over the conventional two-endonuclease method (Figure 2). This approach may be useful for identifying co-dominant genetic markers or differences in closely related genomes, and can easily be applied for characterisation of a variety of taxa.

### Example 5

### Comparison of the four- and two-endonuclease methods

The efficiency of the conventional two-endonuclease method and a method according to the present invention using four endonuclease enzymes for generating additional polymorphisms for finer genotyping was assessed by directly comparing their fingerprint patterns using trypanosome DNA (Fig. 2).

It was shown that for all the (sub)species analysed, samples processed using the four-endonuclease method according to the present invention revealed remarkably more restriction fragments than those analysed with the two-enzyme method.

Moreover, extra polymorphic fragments resulted from the four-endonuclease method, indicating that additional restriction sites were indeed accessed, compared to the conventional two-endonuclease approach.

However, the increase in the number of fingerprint fragments was not linear when compared to the two-endonuclease approach with *Bgl*II and *EcoR*I. In principle, the pair of endonucleases, *Bgl*II (A/GATCT) and *BclI* (T/GATCA) when used together to digest the genome would cleave 6-bp palindromic 5'-GATC- sequences if an A or T is present at the 5' end, respectively.

Assuming both restriction enzyme sites to be equally distributed in the genome and that all sites are cleaved, this would imply twice as much cleavage as with only one of the two enzymes, with a cutting frequency of one every 2048 (4⁶/2) bp in the genome.

In reality, this is highly unlikely due to several factors. First, the recognition sequences of the endonucleases may overlap at certain positions. In this case, depending on the restriction enzyme that cleaves first, the second enzyme may not cut since its recognition sequence would have been disrupted by the previous cleavage. Taking this into account in the simulation studies, a pair of endonucleases that share the same nucleotides at two or four positions (e.g. *Bgl*II/*Bcl*I and *Bgl*II/*EcoR*I), were predicted to give approximately the same mean cutting frequency, 2269 bp and 2275 bp, respectively. However, in a *Bgl*II*-Bcl*I*-Mun*I*-EcoR*I digestion, a site for any of the four restriction enzymes is predicted to occur every 1501 bp.

Secondly, variations from predicted outcomes could also be due, in part, to the differential effects of methylation on enzyme sensitivity to substrate. For example, while both *Bgl*II and *Bcl*I recognition sequences completely overlap Dam methylase site GATC, methylation does not block cleavage with *Bgl*II, but it does block cleavage with *Bcl*I, so that restriction may not occur at all *BclI* recognition sites. Similarly, *EcoR*I recognition site (G/AATTC) is insensitive to methylation, while the cleavage of *Mun*I (C/AATTG) site is completely blocked by Dam-methylation.

Thirdly, a common limitation of ligation-mediated restriction analyses is that, in the ligation step, two juxtaposed 5' phosphate and 3' hydroxyl termini in duplex DNA fragments are covalently joined by DNA ligase. As a result, the actual number of amplified fragments is less than the theoretically amplifiable fragments. Since more restriction fragments are generated in the four-endonuclease approach, this phenomenon would be expanded, and may partly account for the non-linear increase in the number of derivable amplified fragments.

Finally, liberal assumptions have been made that all four restriction enzyme recognition sequences are randomly and equally distributed in the genome, and that all restriction sites are cleaved.

### Example 6

### Trypanosome species: intra- and inter-species analyses of genetic differences

We previously evaluated the conventional two endonuclease method as a tool for generating useful markers for finer characterization of trypanosome isolates to subspecies level (Agbo *et al*., 2002). This method was limited by the fact that only marked size differences based on allelic restriction fragments from two restriction enzymes could be assessed and, as a result, only few polymorphic markers could be detected.

An important issue that arises in the context of using genetic markers for classifying individuals relates to the number of fragments and markers needed to provide adequate fingerprint or clustering. The more restriction sites are accessed in the genome, the more informative the resulting representation fragments in finding polymorphisms.

Therefore, a minimum number of fragments (and markers) are needed for finding the underlying structural patterns of diversity in a population of interest, and using this information to obtain more precise clusters of genotypes.

We have now found a unique multiplex-endonuclease method, as herein described, to expand the number of derivable fingerprint fragments, in combination with a pair of adapters and cognate primers to ensure stringency in PCR amplification.

For the inter- and intra-species analyses of trypanosome isolates (Table 1), the *Bgl*II*-Bcl*I/*EcoR*I*-Mun*I combination was selected on the basis of their reproducibility, even distribution of bands along the gel and number of polymorphic bands detected (Fig. 2, A-F). The intra-specific analyses of *Trypanosoma brucei* subspecies with four endonucleases (A) resulted in more restriction fragments and extra polymorphisms compared to the conventional two-endonuclease method (B).

Similarly, with the closely related *T. evansi* and *T. equiperdum* species (C and D), a higher number of fragments resulted with the present four-endonuclease method, but less so with the number of identifiable polymorphisms between the species. This underscores the close genetic relatedness of the two species, being probably pathotypes of the same parental strain.

The greater discriminatory power of a method according to the invention using four-endonucleases was best demonstrated in the inter-species analyses of *T. congolense* and *T. simiae* isolates, in which far more polymorphic fragments result (E) than with the two-endonuclease approach (F).

These results propose that additional sites within the genome were indeed accessed in the four-endonuclease method. When, for instance, the same region of the genome is accessed in both approaches, there is more coverage with the multi-endonuclease method so that more polymorphic restriction fragments are accessed and detected than with two or three endonucleases.

Nearly all eukaryotes contain non-coding repeat sequences that are under minimal evolutionary pressure. Therefore, since DNA polymorphisms may be concentrated at these regions, they will be highly informative for DNA fingerprinting, genetic mapping and population structure studies. It is thus possible that DNA polymorphisms can be overlooked when only a limited number of restriction enzymes have been tested with the conventional methods.

This might explain the success of the four-endonuclease method as described herein over the conventional two-endonuclease method and underline the greater inter-species genetic difference (Fig. 2 E, F) when compared to differences within the members of the *Trypanozoon* subgenus *(T. brucei, T. evansi* and *T. equiperdum*).

Crossing human serum resistant strains of *T. brucei* with susceptible strains and then assessing the phenotype of the progeny with Blood Incubation Infectivity Test (BIIT) (Rickman and Robson, 1970) has shown that human serum resistance (equivalent to human infectivity) is, at least in part, a dominant trait (Gibson and Stevens, 1999). Since restriction enzyme polymorphisms are dominant markers, the observed polymorphisms among *T. brucei* subspecies support the proposition (Gibson and Stevens, 1999) that the trait of human serum resistance is dominantly controlled.

Secondly, finer genetic differences, i.e. more polymorphic sites, among trypanosome (sub)species can be consistently detected by using additional sets or other combinations of endonucleases selected on the basis of the method described herein. Thereby, unique regions, such as subspecies-specific regions, may be identified to which unique markers, such as subspecies-specific markers may then be designed.

An embodiment according to the present invention wherein four-endonucleases are used has shown to give higher resolution power over the two-endonuclease method, while the use of only one pair of primers in PCR ensures stringency and robustness of the method.

### Conclusions

The present invention permits a more extensive coverage of the genome under study. The complexity of the fingerprint can be advantageously managed for finer genetic analysis by increasing the number and/or varying the choice of restriction enzyme pairs. This principle can be applied for accessing additional polymorphisms in differential display studies.

Furthermore, the present invention is also valuable in genomic DNA or c-DNA subtractive-hybridisation studies where biased amplification of DNA fragments in the initial complex pool favours fragments of smaller size. As a result, the complex pool of products obtained after PCR amplification of the initial sample constitutes a 'representation' of the genome, the complexity of which may be substantially less than that of the original genomic DNA. This is partially compensated for by the use of 4-bp recognising endonucleases. However, the segment of the genome that is accessed is usually dependent upon the recognition sequence of the single endonuclease that is used. As such, the restriction enzyme recognition sequence may be biased in favour of AT- or GC-rich genomic or cDNA regions.

A number of endonuclease combinations selected on the basis of the present invention can be used to analyse additional regions of the genome, and to increase the 'representation' of the generated fragments.

These features make the present invention better suited for revealing higher levels of genetic variation than with previously described methods (Lindstedt *et al*., 2000; Simons *et al*., 1997; Van der Wurff *et al*., 2000). For micro-restriction mapping of *T. brucei* subspecies, additional endonucleases in varying combinations and numbers can now be utilised to directly access more genomic regions for genetic markers that may be associated with human resistance/sensitivity.

### References

Agbo, E.C., Majiwa, P.A.O, Claassen, E.J.H.M. and Roos, M.H. (2001). Measure of molecular diversity within the *Trypanosoma brucei* subspecies *Trypanosoma brucei brucei* and *Trypanosoma brucei gambiense* as revealed by genotypic characterisation. *Exp. Parasitol*., **99**, 123-131

Agbo, E.C., Majiwa, P.A.O., Claassen, E.H.J.M. and Pas, M.F.W. (2002). Molecular variation of *Trypanosoma brucei* subspecies as revealed by AFLP fingerprinting. *Parasitol*. **124**, 000-000.

Barany, F. (1991). Genetic disease detection and DNA amplification using cloned thermostable ligase. *Proc Natl Acad Sci* USA **88(1)**, 189-193.

Gibson, W.C and Stevens, J. (1999). Genetic exchange in *Trypanosomatidae. Advs. in Parasitol*., **43,** 1-45.

Gibson, W.C., Marshall, T.F. de C. and Godfrey, D.G. (1980). Numerical analysis of enzyme polymorphism: a new approach to epidemiology and taxonomy of trypanosomes of the subgenus *Trypanozoon. Adv. in Parasitol*., **18,** 175-246.

Guatelli J.C., Whitfield K.M., Kwoh D.Y., Barringer K.J., Richman D.D. and Gingeras T.R. (1990). Isothermal, in vitro amplification of nucleic acids by a multienzyme reaction modeled after retroviral replication. *Proc Natl Acad Sci* USA **87**(5), 1874-1878.

Hawking, F. (1973). The differentiation of *Trypanosoma rhodesiense* from *T. brucei* by means of human serum. *Transactions of the Royal Soc. of Trop. Med. and Hygiene,* **67**, 517-527.

Hawking, F. (1977). The resistance to human plasma of *Trypanosoma brucei, T. rhodesiense* and *T. gambiense.* In: Analysis of the composition of trypanosome strains. *Transactions of the Royal Soc. of Trop. Med. and Hygiene*, **70**, 504-512.

Heath, S. (1997). Molecular Techniques in Analytical Parasitology. In *Analytical Parasitology* (ed. Rogan, M.T.), pp 67-68.

Kassai, K (1988). Standardized nomenclature of animal parasitic disease (SNOAPAD). *Vet. Parasitol*., **29**, 299-326.

Kramer F.R., Lizardi P.M. (1990). Amplifiable hybridization probes. *Ann Biol Clin* (Paris). **48**(6), 409-11.

Kwoh D.Y., Davis G.R., Whitfield K.M., Chappelle H.L., DiMichele L.J., Gingeras T.R. (1989). Transcription-based amplification system and detection of amplified human immunodeficiency virus type 1 with a bead-based sandwich hybridization format. *Proc Natl Acad Sci* USA **86**(4), 1173-1177.

Lindstedt, B-A,. Heir, E., Vardund, T. and Kapperd, G. (2000). A variation of the amplified fragment length polymorphism (AFLP) technique using three restriction endonucleases, and assessment of the enzyme combination *Bgl*II*-Mfe*I for AFLP analysis of *Salmonella enterica* subsp. *enterica* isolates. *FEMS*

Lizardi P.M., Huang, X.,Zhu, Z., Bray-Ward, P., Thomas, D.C. and Ward, D.C. (1998). Mutation detection and single-molecule counting using isothermal rolling-circle amplification. *Nature Genetics* 19:225-232.

*Microbiol. Letts.,* **189**,19-24.

Mueller, U.G. and Wolfenbarger, L.L. (1999). AFLP genotyping and fingerprinting - a review. *Trends in Ecol. & Evol.,* **14**, 389-394.

Mullis, K.B., and Faloona, F.A. (1987). Specific synthesis of DNA in vitro via a polymerase-catalyzed chain reaction. *Methods Enzymol*., **155**, 335-50.

Pearson, K. (1926). On the coefficient of racial likeness. *Biometrika*, **18**, 105-117.

Rickman, L.R and Robson, J. (1970). The testing of proven *Trypanosoma brucei* and *T. rhodesiense* strains by the blood incubation infectivity test. *Bulletin of the World Health Organisation* **42**, 911-916.

Simons, G., van der Lee, T., Diergaarde, P., van Daelen, R., Groenendijk, J., Frijters, A., Buschges, R., Hollricher, K., Topsch, S., Schulze-Lefert, P., Salamini, F., Zabeau, M. and Vos, P. (1997). AFLP-based fine mapping of the Mlo gene to a 30-kb DNA segment of the barley genome. *Genomics*, **44**, 61-70.

Van der Wurff, A.W.G., Chan, Y.L., van Straalen, N.M. and Schouten, J. (2000). TE-AFLP: combining rapidity and robustness in DNA fingerprinting. *Nucleic Acids Res.,* **28**, e105.

Vauterin, L.A. and Vauterin, P. (1992). Computer-aided objective comparison of electrophoresis patterns for grouping and identification of microorganisms. *European Microbiol. J*., **1**, 37-41.

Vos, P., Hogers, M., Bleeker, M., Reijans, M., van de Lee, T., Hornes, M., Frijters, A., Pot, J., Peleman, J., Kuiper, M. and Zabeau, M. (1995). AFLP: a new technique for DNA fingerprinting. *Nucleic Acids Res.,* **23,** 4407-4414.

## Claims

1. Method for producing a nucleic acid fingerprint comprising the steps of:
(a) providing from a starting nucleic acid a plurality of adapter ligatable nucleic acid fragments with ends that are compatible to at least one adapter, and wherein said nucleic acid fragments are obtained by fragmentation of said starting nucleic acid;
(b) performing a ligation reaction between said ends of said nucleic acid fragments and said at least one adapter such as to produce adapter-ligated nucleic acid fragments;
(c) amplifying said adapter-ligated nucleic acid fragments by using at least one amplification primer essentially complementary to the nucleotide sequence of said at least one adapter; and
(d) generating from said amplified adapter-ligated nucleic acid fragments a nucleic acid fingerprint.

2. Method according to claim 1, wherein said nucleic acid fragments are obtained by specific fragmentation of said starting nucleic acid.

3. Method according to claim 2, wherein said specific fragmentation comprises the use of at least two different restriction endonuclease enzymes that produce restriction fragments with ends that are compatible to a single adapter.

4. Method according to claim 2, wherein said specific fragmentation comprises the use of at least three different restriction endonuclease enzymes, at least two of which enzymes produce restriction fragments with ends that are compatible to a single adapter.

5. Method according to claim 2, wherein said specific fragmentation comprises the use of at least four different restriction endonuclease enzymes, of which enzymes at least two produce restriction fragments with ends that are compatible to a first adapter.

6. Method according to claim 5, wherein said at least four different restriction endonuclease enzymes comprise at least a further two enzymes that produce restriction fragments with ends that are compatible to a second adapter.

7. Method according to any one of the claims 3-6, wherein said compatible ends comprise cohesive ends.

8. Method according to any one of the claims 3-7, wherein said restriction endonuclease enzymes are 4 to 8 bp-cutting restriction endonuclease enzymes.

9. Method according to any one of the claims 3-8, wherein said restriction endonuclease enzymes that produce restriction fragments with compatible ends are selected from Table 3.

10. Method according to claim 5 or 6, wherein said at least four different restriction endonuclease enzymes are chosen from the group consisting of (i) *Bgl*II, *Bcl*I, *EcoR*I and *Mun*I; (ii) *Bgl*II, *Bcl*I, *Acs*I and *Mun*I; (iii) *Bcl*I, *Xho*II, *Acs*I and *Mun*I; and (iv) *Bgl*II, *Xho*II, *EcoR*I and *Acs*I.

11. Method according to claim 10, wherein said first and second adapter are a *Bgl*II and a *Mun*I adapter.

12. Method according to any one of the preceding claims, wherein step b) comprises the formation of heterosite nucleic acid fragments.

13. Method according to any one of the preceding claims, wherein step c) comprises the use of at least one primer that comprises selective nucleotides.

14. Method according to any one of the preceding claims, wherein step d) comprises the use of electrophoresis.

15. Method according to claim 14, further comprising the use of fluorimetry, autoradiography, phospho-imaging, or other methods for visualizing nucleic acid fingerprints.

16. Method according to any one of the preceding claims wherein said nucleic acid is genomic DNA.

17. Method for detecting sequence polymorphisms between one or more genomes comprising producing a nucleic acid fingerprint from said genomes by using a method according to any one of the preceding claims and comparing the obtained nucleic acid fingerprints for the presence or absence of, or differences between, amplified nucleic acid fragments such as to determine the presence of sequence polymorphisms.

18. Method according to claim 17, wherein said genomes are the genomes from lower level taxa.

19. Method according to claim 18, wherein said lower level taxa comprise species, subspecies, strains, variatas, forms, cultivars or sub-populations.

20. Method according to any one of claims 17-19, wherein said genomes are the genomes from parasites.

21. Method for the identification of DNA markers linked to a specific phenotype, a phenotypic characteristic and/or a genetic trait, comprising detecting polymorphisms by using a method according to any one of the claims 17-20, between starting nucleic acids origination from organisms which exhibit differences in said specific phenotype, phenotypic characteristic and/or genetic trait and correlating said polymorphisms to said differences.

22. Method according to claim 21, wherein said DNA markers comprise SNP markers, multi-locus markers, single locus markers, dominant markers, diagnostic markers, phenotypic markers, clonal markers and/or reference markers

23. Method for the identification of an unknown organism, comprising preparing a nucleic acid fingerprint from said unknown organism by using a method according to any one of the claims 1-16, comparing said fingerprint with at least one nucleic acid fingerprint prepared from at least one known organism by that same method and establishing the identity of said unknown organism on the basis of similarity of said nucleic acid fingerprint from said unknown organism with said at least one nucleic acid fingerprint from said at least one known organism.

24. A kit for use in a method according to any of the claims 1-23, which kit comprises one or more elements selected from the group consisting of restriction endonuclease enzymes, buffers, double stranded DNA adapters, DNA ligase enzymes, cognate amplification primers optionally labeled with a suitably detectable label, DNA polymerase enzymes, reference DNA and data analysis software.
